(19) Europäisches Patentamt
European Patent Office
Office européen des brevets

(11) Numéro de publication : **0 035 927**
**B1**

# (12) FASCICULE DE BREVET EUROPÉEN

(45) Date de publication du fascicule du brevet : **26.01.83**

(51) Int. Cl.³ : **C 07 C 43/225**, C 07 C 41/16

(21) Numéro de dépôt : **81400304.2**

(22) Date de dépôt : **27.02.81**

## (54) Procédé de préparation de chloroalkoxybenzènes.

(30) Priorité : **12.03.80 FR 8005489**

(43) Date de publication de la demande : **16.09.81 Bulletin 81/37**

(45) Mention de la délivrance du brevet : **26.01.83 Bulletin 83/04**

(84) Etats contractants désignés : **AT BE CH DE FR GB IT LI LU NL SE**

(56) Documents cités : **CHEMICAL ABSTRACTS, vol. 87, 1977 abrégé no. 22645n Columbus, Ohio US S. M. SHEIN et al. : « Synthesis of polyphenyl ethers from dihydric phenols ».**

(73) Titulaire : **RHONE-POULENC SPECIALITES CHIMIQUES**
**"Les Miroirs" 18, Avenue d'Alsace**
**F-92400 Courbevoie (FR)**

(72) Inventeur : **Soula, Gérard**
**33, rue Nungesser**
**F-69330 Meyzieu (FR)**
Inventeur : **Linguenheld, Louis**
**6, rue d'Illzach**
**F-68270 Ruelisheim (FR)**

(74) Mandataire : **Cazes, Jean-Marie et al**
**RHONE-POULENC RECHERCHES Service Brevets Chimie et Polymères 25, quai Paul-Doumer**
**F-92408 Courbevoie Cedex (FR)**

EP 0 035 927 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

Procédé de préparation de chloroalkoxybenzènes

La présente invention a pour objet un procédé de préparation de chloroalkoxybenzènes ; elle concerne plus particulièrement la préparation de chloroalkoxybenzènes à partir de chlorobenzènes.

On connaît dans l'art antérieur un certain nombre de procédés de ce type.

C'est ainsi que la réaction du méthylate de sodium sur le trichloro-1,2,3 benzène en solution dans le méthanol à 180 °C sous pression est décrite (Recueil des Travaux Chimiques des Pays-Bas *37* 200) pour l'obtention du dichloro-2,3 anisole. Ce procédé ne peut être retenu pour une exploitation industrielle dans la mesure où il donne naissance à de nombreux sous produits et où les rendements obtenus sont très faibles.

On connaît également le brevet des Etats-Unis d'Amérique 4057585 qui décrit la préparation de dichloro-2,3 alkoxy-1 benzène par réaction d'un alcoolate alcalin et de trichloro-1,2,3 benzène à 100-200 °C dans un solvant inerte choisi parmi le groupe comprenant le diméthylformamide, le diméthylacétamide et le diméthylsulfoxyde, la réaction ayant lieu en présence de méthanol ou d'éthanol en quantité suffisante pour solvater l'alcoolate alcalin. Dans ce type de procédé, deux inconvénients majeurs rendent son exploitation industrielle difficile. Le premier réside dans le fait qu'il est nécessaire d'utiliser un alcoolate alcalin parfaitement anhydre, donc préparé à partir de l'alcalin correspondant sous sa forme métallique. En effet, il faut utiliser un alcoolate exempt de soude sinon cette dernière dégraderait le solvant mis en œuvre. Le deuxième inconvénient tient au fait que les solvants préconisés sont, d'une part, onéreux et, d'autre part, de manipulation malaisée à l'échelle industrielle lors des opérations nécessaires de distillation et recyclage, notamment.

D.J. SAM et H.E. SIMMONS décrivent dans Journal of the American Chemical Society Vol. 96 n° 17 p. 2252 (1974), la réaction de l'o. ou m. dichlorobenzène avec du méthylate de potassium complexé par une quantité équimoléculaire d'un éther-couronne (dicyclohexyl-18 crown-6). Cette technique n'est pas bénéfique, d'une part, au plan de l'économie du procédé et, d'autre part, au plan de sa simplicité de mise en œuvre. En effet, les « éthers couronnes » sont des composés dont le prix de revient est très élevé. De plus, il est nécessaire de préparer dans une étape préliminaire le complexe éther-couronne-alcoolate.

La présente invention pallie les inconvénients de l'art antérieur. Elle a pour objet un procédé de préparation de chloroalkoxybenzènes, caractérisé en ce que l'on fait réagir un polychlorobenzène de formule générale :

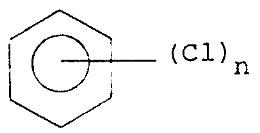 (I)

dans laquelle n est supérieur ou égal à 2 et inférieur ou égal à 6 ($2 \leq n \leq 6$) avec, au moins un hydroxyde alcalin et un alcanol de formule ROH (II) dans laquelle R contient de 1 à 12 atomes de carbone en présence d'un agent séquestrant de formule générale :

$$N[-CHR_1-CHR_2-O-(CHR_3-CHR_4-O)_n-R_5]_3 \quad (III)$$

dans laquelle n est un nombre entier supérieur ou égal à 0 et inférieur ou égal à 10 ($0 \leq n \leq 10$), $R_1$, $R_2$, $R_3$, $R_4$ identiques ou différents représentent un atome d'hydrogène ou un radical alkyle ou cycloalkyle ayant de 1 à 12 atomes de carbone, un radical phényle ou un radical de formule $-C_mH_{2m}-\emptyset$, ou $C_mH_{2m+1}-\emptyset-$, m étant compris entre 1 et 12.

Bien que cela ne soit pas clairement et totalement établi, il semble que l'agent séquestrant de formule (III) forme avec l'alcoolate alcalin résultant de la réaction de ROH avec l'hydroxyde alcalin, un complexe soluble dans le dérivé benzénique de formule I. La réaction peut, de ce fait, avoir lieu sans solvant.

Selon un mode de réalisation préférentiel de l'invention, on introduit dans le mélange polychlorobenzène-agent séquestrant(s), le mélange hydroxyde(s) alcalin(s)-alcool ROH, l'alcoolate alcalin intervenant dans la réaction étant généré in situ. Un des avantages principaux du procédé selon l'invention ressort clairement de ce qui précède dans la mesure où il n'est pas nécessaire comme dans l'art antérieur, soit d'utiliser l'alcalin sous sa forme métallique, soit d'isoler dans une étape préliminaire le complexe alcoolate alcalin-agent séquestrant.

Selon un mode de réalisation préférentiel du procédé de l'invention, on utilise au moins un agent séquestrant de formule (III) dans laquelle $R_1$, $R_2$, $R_3$ et $R_4$ représentent un atome d'hydrogène ou un radical méthyle, $R_5$ et n ayant la signification précédente.

Parmi ces derniers, on préfère encore plus particulièrement mettre en œuvre les agents séquestrants pour lesquels n est supérieur ou égal à 0 et inférieur ou égal à 6, et pour lesquels $R_5$ représente un radical alkyle ayant de 1 à 4 atomes de carbone.

On peut citer :

— la tris(oxa-3 bytyl)amine de formule :

$N—(CH_2—CH_2—O—CH_3)_3$
- la tris(oxa-3 heptyl)amine de formule : $N—(CH_2—CH_2—O—C_4H_9)_3$
- la tris(dioxa-3,6 heptyl)amine de formule : $N—(CH_2—CH_2—O—CH_2—CH_2—O—CH_3)_3$
- la tris(trioxa-3,6,9 décyl)amine de formule : $N—(CH_2—CH_2—O—CH_2—CH_2—O—CH_2—CH_2—O—CH_3)_3$
- la tris(dioxa-3,6 octyl)amine de formule : $N—(CH_2—CH_2—O—CH_2—CH_2—O—C_2H_5)_3$
- la tris(trioxa-3,6,9 undécyl)amine de formule : $N—(CH_2—CH_2—O—CH_2—CH_2—O—CH_2—CH_2—O—C_2H_5)_3$
- la tris(dioxa-3,6 monyl)amine de formule : $N—(CH_2—CH_2—O—CH_2—CH_2—O—C_3H_7)_3$
- la tris(trioxa-3,6,9 dodécyl)amine de formule : $N—(CH_2—CH_2—O—CH_2—CH_2—O—CH_2—CH_2—O—C_3H_7)_3$
- la tris(dioxa-3,6 décyl)amine de formule : $N—(CH_2—CH_2—O—CH_2—CH_2—O—C_4H_9)_3$
- la tris(trioxa-3,6,9 tridécyl)amine de formule : $N—(CH_2—CH_2—O—CH_2—CH_2—O—CH_2—CH_2—O—C_4H_9)_3$
- la tris(oxa-3 butyl)amine de formule : $N—(CH_2—CH_2—O—CH_3)_3$
- la tris(tétraoxa-3,6,9,12 tridécyl)amine de formule : $N—[CH_2—CH_2—O—(CH_2—CH_2—O—)_3—CH_3]_3$
- la tris(hexaoxa-3,6,9,12,15,18 nonadécyl)amine de formule : $N—[CH_2—CH_2—O—(CH_2—CH_2—O)_5CH_3]_3$

On peut encore citer :

- la tris(dioxa-3,6 méthyl-4 heptyl)amine de formule : $N—(CH_2—CH_2—O—CHCH_3—CH_2—O—CH_3)_3$
- la tris(dioxa-3,6 diméthyl-2,4 heptyl)amine de formule : $N—(CH_2—CHCH_3—O—CHCH_3—CH_2—O—CH_3)_3$.

Les amines utilisées dans le procédé selon l'invention sont connues en tant que telles dans l'art antérieur. C'est ainsi que le brevet français 1 302 365 cite l'obtention des amines tertiaires $N—(CH_2—CH_2—O—CH_3)_3$ et $N—(CH_2—CH_2—O—CH_2—CH_2—O—CH_3)_3$ comme sous produits de la synthèse des amines primaires et secondaires correspondantes, ces amines primaires et secondaires étant des produits intéressants comme intermédiaires en vue de la synthèse de substances pharmaceutiques, comme inhibiteurs de corrosion, comme intermédiaires en vue de la synthèse de produits chimiques intéressants en agriculture et comme émulsifiants. Il n'est pas inutile de souligner que le domaine d'application des composés obtenus dans le brevet 1 302 365 précité simultanément aux amines utilisées dans le procédé objet de la présente demande est totalement étranger au domaine de l'invention.

L'hydroxyde alcalin est choisi préférentiellement parmi le groupe comprenant la soude et la potasse.

On préfère plus particulièrement mettre en œuvre la potasse ou un mélange soude-potasse. Dans ce dernier cas, on utilise préférentiellement un mélange contenant au moins 50 % de potasse.

On utilise de préférence, l'agent séquestrant de formule (III) en quantité telle que le rapport molaire de l'agent séquestrant à l'hydroxyde alcalin est compris entre 0,001 et 0,2. Ce rapport est, encore plus préférentiellement, compris entre 0,01 et 0,1.

On peut citer comme exemples de composés de départ de formule (I), les composés suivants : l'orthodichlorobenzène, le métadichlorobenzène, le paradichlorobenzène, le trichloro-1,2,4 benzène, le trichloro-1,2,3 benzène, le trichloro-1,3,5 benzène, le tétrachlorobenzène, le pentachlorobenzène et l'hexachlorobenzène.

Il est possible de mettre en œuvre la présente invention en utilisant des polychlorobenzènes contenant de 2 à 5 atomes de chlore et comportant en outre au moins un substituant ne réagissant pas chimiquement dans le procédé selon l'invention.

On peut citer comme exemples non limitatifs de tels substituants : les radicaux alkyle, phényle, $NO_2$. Ce dernier peut dans certains cas être intéressant dans la mesure où il active le noyau benzénique.

On peut citer comme exemples de composés de formule II pouvant être mis en œuvre dans le procédé de l'invention : le méthanol, l'éthanol, l'isopropanol, les butanols, le cyclohexanol, l'octanol.

On peut citer comme exemples de chloroalkoxybenzènes pouvant être préparés par le procédé de l'invention les composés suivants : l'ortho-méta ou para chloroanisole, le dichloro-2,3 anisole, le dichloro-2,6 anisole, le dichloro-2,5 anisole, le trichloro-2,4,5 anisole, le trichloro-2,3,6 anisole, le tétrachloro-2,3,4,6 anisole, le pentachloroanisole, le dichloro-2,3 éthoxybenzène, le dichloro-2,5 éthoxybenzène, le dichloro-2,5 isopropoxybenzène.

On utilise de préférence les composés I et l'hydroxyde alcalin en quantité telle que le rapport molaire

de l'hydroxyde alcalin au composé I est compris entre 0,5 et 3. Encore plus préférentiellement, ce rapport est compris entre 0,8 et 2,5.

La quantité d'alcool peut varier dans de larges limites. On utilise de préférence une quantité d'alcool telle que le rapport molaire de ROH à l'hydroxyde alcalin est compris entre 1 et 15. Encore plus préférentiellement, ce rapport est compris entre 6 et 8.

La réaction peut être mise en œuvre à une température comprise entre 80 °C et 250 °C. Plus particulièrement, on préfère opérer à une température comprise entre 100 et 180 °C.

On opère généralement à la pression atmosphérique, bien que des pressions supérieures ou inférieures à la pression atmosphérique ne soient pas exclues.

Bien que cela ne soit pas nécessaire, on peut utiliser un tiers solvant, comme par exemple le toluène, l'o-, p- ou m-xylène, ou le chlorobenzène.

Les agents séquestrants de formule III utilisés dans le procédé selon l'invention peuvent être préparés par condensation d'un sel de formule :

$$R_5—(O—CHR_4—CH_3)_n—O—M$$

où $R_3$, $R_4$, $R_5$, et n ont la signification précédente et où M représente un atome de métal alcalin choisi parmi le sodium, le potassium et le lithium, soit sur une amine de formule générale :

$$N—(CHR_1—CHR_2—X)_3$$

dans laquelle $R_1$, et $R_2$ ont la signification précédente et X représente le chlore ou le brome, soit sur le chlorhydrate ou le bromhydrate correspondant.

Le rapport molaire sel de métal alcalin/amine est compris entre environ 3 et environ 5.

L'opération de condensation est réalisée à une température comprise entre 100 et 150 °C pendant 1 à 15 heures en présence d'un solvant qui peut être par exemple le chlorobenzène ou de préférence le monoalkyléther d'éthylène glycol de formule :

$$R_5—(O—CHR_4—CHR_3)_n—OH.$$

On opère de préférence de telle sorte qu'on ait une solution contenant de 2 à 5 moles de sel de métal alcalin par litre de solvant.

Le mélange en fin de réaction contient principalement l'amine tertiaire de formule :

$$N—[CHR_1—CHR_2—O—(CHR_3—CHR_4—O)_nR_5]_3$$

mais contient aussi en faible proportion de l'amine secondaire correspondant :

$$HN—[CHR_1—CHR_2—O—(CHR_3—CHR_4—O)_n—R_5]_2$$

et des traces d'amine primaire :

$$H_2N—[CHR_1—CHR_2—O—(CHR_3—CHR_4—O)_n—R_5].$$

Les amines tertiaires, secondaires et primaires sont généralement respectivement dans le rapport 90 : 8 : 2 après distillation.

On peut utiliser dans le procédé selon l'invention directement le mélange ci-dessus obtenu après première distillation, c'est-à-dire contenant les trois types d'amines.

On préfère pour une meilleure mise en œuvre de l'invention effectuer une distillation plus poussée du mélange ci-dessus afin d'obtenir une amine tertiaire sensiblement pure.

Les exemples qui vont suivre illustrent plus complètement les caractéristiques et les avantages de la présente invention mais ne sauraient être considérés comme limitant de façon quelconque cette dernière.

## Exemple 1

Préparation de dichloro-2,3 anisole à partir de trichlorobenzène

Dans un ballon tricol de 6 litres équipé d'une agitation, d'un thermomètre, d'une ampoule de coulée et d'une colonne à distiller, on charge :

trichloro 1,2,3 benzène 1 815 g (10 moles)
tris(dioxa 3,6 heptyl)amine 162 g (0,5 mole).

On chauffe la masse à 140° et on commence la coulée d'une solution méthanolique de potasse préalablement préparée à partir de :

| | | |
|---|---|---|
| 1 302,5 g | potasse pastilles 86 % | (20 moles) |
| 2 500 g | méthanol | (78,1 moles). |

La coulée et le chauffage sont réglés de façon à maintenir 135° dans la masse pendant que le méthanol distille. La coulée de la potasse dure 1 h 15 puis on maintient la masse à 135° pendant 1 h 15 de finition.

Après refroidissement, on ajoute 2 800 g d'eau pour extraire les sels. On décante la couche organique, extrait la couche aqueuse avec 1 000 g de dichlorométhane. Après séchage, la couche organique est distillée pour obtenir :

| | | |
|---|---|---|
| 100 g | de 1,2,3 trichlorobenzène | (0,55 mole) |
| 454 g | de 2,6 dichloroanisole | (2,565 moles) |
| 700 g | de 2,3 dichloroanisole | (3,955 moles). |

Le taux de transformation du trichlorobenzène est alors de 93,15 % et le rendement en dichloro-2,3 anisole atteint 39,5 %. Par acidification de la couche aqueuse, on récupère 342 g de dichlorophénols, principalement constitué de 2,6 dichlorophénol.

## Exemple 2

Dans l'appareillage décrit à l'exemple 1, on charge :

| | | |
|---|---|---|
| trichloro-1,2,3 benzène | 363 g | (2 moles) |
| tris(dioxa-3,6 heptyl)amine | 32 g | (0,1 mole). |

On chauffe ce mélange à 130 °C et on coule en 1 h 30 une solution méthanolique de soude et de potasse préalablement préparée en dissolvant

| | | | |
|---|---|---|---|
| 117,2 g | potasse | 86 % | (1,8 mole) |
| 74,2 g | soude | 97 % | (1,8 mole) |

dans 800 g de méthanol.

Le méthanol est distillé au fur et à mesure de la coulée.

Après l'addition, on maintient la masse à 130-140 °C pendant 3 h 30.

Après refroidissement, la masse réactionnelle est alors traitée comme dans l'exemple 1.

Le taux de transformation atteint 84,5 % et le rendement en 2,3-dichloroanisole est de 39 % et dichloro-2,6 anisole de 37 %.

## Exemple 3

Dans l'appareillage, décrit dans l'exemple 1, et suivant, le même mode opératoire. On engage :

| | | |
|---|---|---|
| trichloro-1,2,3 benzène | 363 g | (2 moles) |
| tris(trioxa-3,6,9 décyl)amine | 42 g | (0,09 mole) |

et une solution méthanolique de potasse préalablement préparée par dissolution de :

potasse 86 % 234,4 g (3,6 moles)

dans 400 g de méthanol.

Après 3 h de maintien à 125 °C, la masse est traitée comme précédemment.

Le taux de transformation atteint 94,8 % et le rendement en 2,3-dichloroanisole est de 42 % et en dichloro-2,6 anisole de 37 %.

## Exemple 4

Dans l'appareillage décrit dans l'exemple 1 et selon le même mode opératoire, on met en jeu :

| | | |
|---|---|---|
| trichloro-1,2,3 benzène | 363 g | (2 moles) |
| tris(dioxa-3,6 heptyl)amine | 32 g | (0,1 mole) |

et une solution méthanolique de soude et de potasse préalablement préparée par dissolution de :

156,3 g de potasse 86 %, soit 2,4 moles

49,5 g de soude 97 %, soit 1,2 mole

dans 610 g de méthanol.

Après 4 h de maintien à 125 °C la masse réactionnelle est traitée comme dans l'exemple 1.

Le taux de transformation atteint 94,1 % et le rendement en 2,3-dichloroanisole est de 38 % et en dichloro-2,6 anisole de 35 %.

Exemple 5

Dans l'appareillage décrit précédemment, on met en œuvre

| | | |
|---|---|---|
| trichloro-1,2,3 benzène | 363 g | (2 moles) |
| tris(dioxa-3,6 heptyl)amine | 32 g | (0,1 mole). |

On chauffe à 140 °C et on coule une solution butanolique de potasse préalablement préparée par dissolution de :

248 g de potasse 86 %, soit 3,80 moles

dans 741 g de butanol normal 10 moles.

Après la fin de coulée, la masse réactionnelle est agitée 5 h à 140-145 °C.

Après un traitement analogue à celui de l'exemple 1, on récupère par distillation :

| | | | |
|---|---|---|---|
| 221 g | dichloro-2,6 butoxy benzène | (1,01 mole) | $Eb_{16}$ : 140° |
| 133,7 g | dichloro-2,3 butoxy benzène | (0,615 mole) | $Eb_{16}$ : 147°. |

Le taux de transformation est de 94,5 % et les rendements sont respectivement de 53,4 % en isomère dichloro-2,6 butoxy benzène et 32,3 % en isomère dichloro-2,3 butoxy benzène.

Exemple 6 :

Préparation de dichloro-2,3 anisole à partir de trichloro-1,2,3 benzène

Dans un ballon tricol de 2 litres équipé d'une agitation, d'un thermomètre et d'une colonne à distiller, on charge :

| | | | |
|---|---|---|---|
| trichloro 1,2,3 benzène | 544,5 g | 3 | moles |
| méthanol | 856 g | 26,75 | moles |
| tris(dioxa 3,6 heptyl)amine | 48 g | 0,148 | mole |
| potasse à 86 % | 195 g | 3 | moles. |

On porte à ébullition et on recueille le méthanol jusqu'à obtention d'une température de 140-150 °C dans la masse. La masse est alors semi-cristallisée pâteuse et la réaction a duré 2 h 30.

Après refroidissement, on ajoute 900 g d'eau pour dissoudre les sels et on décante la couche organique. La couche aqueuse est extraite par 200 g de chlorure de méthylène.

Les couches organiques sont réunies et distillées pour obtenir :

200 g de trichlorobenzène non réagi 1,1 mole
131 g de 2,6 dichloroanisole 0,74 mole

et

142 g de 2,3 dichloroanisole 0,80 mole

La tris(dioxa 3,6 heptyl)amine se trouve intacte dans le pied de distillation.

Dans cet essai, le taux de transformation est de 63 % et le rendement en dichloro 2,3 anisole atteint 42,5 %.

**Revendications**

1. Procédé de préparation de chloroalkoxybenzènes caractérisé en ce que l'on fait réagir un polychlorobenzène de formule générale :

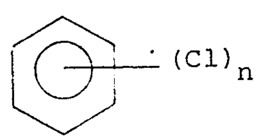

(I)

dans laquelle n est supérieur ou égal à 2 et inférieur ou égal à 6 avec au moins un hydroxyde alcalin et un alcanol de formule ROH (II) ou R contient de 1 à 12 atomes de carbone en présence d'un agent séquestrant de formule générale :

$$N—[CHR_1—CHR_2—O—(CHR_3—CHR_4—O—)_n—R_5]_3. \quad (III)$$

dans laquelle n est un nombre entier supérieur ou égal à 0 et inférieur ou égal à 10 ($0 \leqslant n \leqslant 10$), $R_1$, $R_2$, $R_3$, $R_4$ identiques ou différents représentent un atome d'hydrogène ou un radical alkyle ayant de 1 à 4 atomes de carbone, $R_5$ représente un radical alkyle ou cycloalkyle ayant de 1 à 12 atomes de carbone, un radical phényle ou un radical de formule : $—C_mH_{2m}—Ø$ ou $C_mH_{2m+1}—Ø—$, m étant compris entre 1 et 12.

2. Procédé selon la revendication 1 caractérisé en ce que dans la formule (III) de l'agent séquestrant, $R_1$, $R_2$, $R_3$ et $R_4$ identiques ou différents représentent un atome d'hydrogène ou un radical méthyle.

3. Procédé selon la revendication 1 caractérisé en ce que dans la formule (III) de l'agent séquestrant, n est supérieur ou égal à 0 et inférieur ou égal à 6.

4. Procédé selon la revendication 1 caractérisé en ce que dans la formule (III) de l'agent séquestrant, $R_5$ représente un radical alkyle ayant de 1 à 4 atomes de carbone.

5. Procédé selon la revendication 1 caractérisé en ce que dans la formule (III) de l'agent séquestrant, $R_1$, $R_2$, $R_3$ et $R_4$ identiques ou différents représentent un atome d'hydrogène ou un radical méthyle, n est un nombre entier supérieur ou égal à 0 et inférieur ou égal à 6 et $R_5$ représente un radical alkyle ayant de 1 à 4 atomes de carbone.

6. Procédé selon la revendication 5 caractérisé en ce que l'agent séquestrant de formule (III) est la tris(dioxa-3,6,heptyl)amine de formule :

$$N—(CH_2—CH_2—O—CH_2—O—CH_3)_3.$$

7. Procédé selon la revendication 5 caractérisé en ce que l'agent séquestrant de formule III est la tris(trioxa-3,6,9 décyl)amine.

8. Procédé selon la revendication 1 caractérisé en ce que l'alcanol est choisi parmi le groupe comprenant le méthanol, l'éthanol, l'isopropanol, les butanols, le cyclohexanol, et l'octanol.

9. Procédé selon la revendication 1 caractérisé en ce que le polychlorobenzène de formule I est choisi parmi le groupe comprenant l'orthodichlorobenzène, le métadichlorobenzène, le paradichlorobenzène, le trichloro-1,2,4 benzène, le trichloro-1,2,3 benzène, le trichloro-1,3,5 benzène, le tétrachlorobenzène, le pentachlorobenzène et l'hexachlorobenzène.

10. Procédé selon la revendication 1 caractérisé en ce que l'hydroxyde alcalin est la soude ou la potasse.

11. Procédé selon la revendication 1 caractérisé en ce que l'on utilise un mélange de soude et de potasse contenant au moins 50 % de potasse.

12. Procédé selon la revendication 1 caractérisé en ce que l'on fait réagir le composé I et l'hydroxyde alcalin en quantités telles que le rapport molaire de l'hydroxyde alcalin au composé I est compris entre 0,5 et 3.

13. Procédé selon la revendication 12, caractérisé en ce que le rapport molaire est compris entre 0,8 et 2,5.

14. Procédé selon la revendication 1 caractérisé en ce que l'on fait réagir l'alcool de formule I en quantité telle que le rapport molaire de l'alcool de formule I à l'hydroxyde alcalin est compris entre 1 et 15.

15. Procédé selon la revendication 14 caractérisé en ce que le rapport molaire est compris entre 6 et 8.

16. Procédé selon l'une quelconque des revendications précédentes caractérisé en ce qu'on opère à une température comprise entre 80 °C et 250 °C.

**Claims**

1. Process for the preparation of chloroalkoxybenzenes, characterised in that a polychlorobenzene of the general formula :

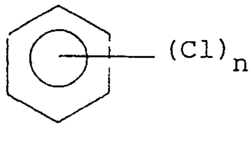

(I)

in which n is greater than or equal to 2 and less than or equal to 6, is reacted with at least one alkali metal hydroxide and an alkanol of the formula ROH (II), in which R contains from 1 to 12 carbon atoms, in the

presence of a sequestering agent of the general of formula :

$$N—[CHR_1—CHR_2—O—(CHR_3—CHR_4—O—)_n—R_5]_3. \qquad (III)$$

in which n is a whole number which is greater than or equal to 0 and less than or equal to 10 ($0 \leq n \leq 10$), $R_1$, $R_2$, $R_3$ and $R_4$, which are identical or different, represent a hydrogen atom or an alkyl radical having from 1 to 4 carbon atoms, and $R_5$ represents an alkyl or cycloalkyl radical having from 1 to 12 carbon atoms, a phenyl radical or a radical of the formula : $—C_mH_{2m}—Ø$ or $C_mH_{2m+1}—Ø—$, m being between 1 and 12.

2. Process according to Claim 1, characterised in that, in the formula (III) of the sequestering agent, $R_1$, $R_2$, $R_3$ and $R_4$, which are identical or different, represent a hydrogen atom or a methyl radical.

3. Process according to Claim 1, characterised in that, in the formula (III) of the sequestering agent, n is greater than or equal to 0 and less than or equal to 6.

4. Process according to Claim 1, characterised in that, in the formula (III) of the sequestering agent, $R_5$ represents an alkyl radical having from 1 to 4 carbon atoms.

5. Process according to Claim 1, characterised in that, in the formula (III) of the sequestering agent, $R_1$, $R_2$, $R_3$ and $R_4$, which are identical or different, represent a hydrogen atom or a methyl radical, n is a whole number which is greater than or equal to 0 and less than or equal to 6, and $R_5$ represents an alkyl radical having from 1 to 4 carbon atoms.

6. Process according to Claim 5, characterised in that the sequestering agent of the formula (III) is tris-(3,6-dioxaheptyl)-amine of the formula :

$$N—(CH_2—CH_2—O—CH_2—CH_2—O—CH_3)_3.$$

7. Process according to Claim 5, characterised in that the sequestering agent of the formula III is tris-(3,6,9-trioxadecyl)-amine.

8. Process according to Claim 1, characterised in that the alkanol is chosen from amongst the group comprising methanol, ethanol, isopropanol, butanols, cyclohexanol and octanol.

9. Process according to Claim 1, characterised in that the polychlorobenzene of the formula I is chosen from amongst the group comprising ortho-dichlorobenzene, meta-dichlorobenzene, para-dichlorobenzene, 1,2,4-trichlorobenzene, 1,2,3-trichlorobenzene, 1,3,5-trichlorobenzene, tetrachlorobenzene, pentachlorobenzene and hexachlorobenzene.

10. Process according to Claim 1, characterised in that the alkali metal hydroxide is sodium hydroxide or potassium hydroxide.

11. Process according to Claim 1, characterised in that a mixture of sodium hydroxide and potassium hydroxide is used which contains at least 50 % of potassium hydroxide.

12. Process according to Claim 1, characterised in that the compound I and the alkali metal hydroxide are reacted in amounts such that the molar ratio of the alkali metal hydroxide to the compound I is between 0.5 and 3.

13. Process according to Claim 12, characterised in that the molar ratio is between 0.8 and 2.5.

14. Process according to Claim 1, characterised in that the alcohol of the formula II is reacted in an amount such that the molar ratio of the alcohol of the formula II to the alkali metal hydroxide is between 1 and 15.

15. Process according to Claim 14, characterised in that the molar ratio is between 6 and 8.

16. Process according to any one of the preceding claims, characterised in that the reaction is carried out at a temperature of between 80 °C and 250 °C.

**Ansprüche**

1. Verfahren zur Herstellung von Chloralkoxybenzolen, dadurch gekennzeichnet, daß man ein Polychlorbenzol der allgemeinen Formel

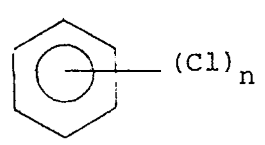

(I)

in der n größer oder gleich 2 und kleiner oder gleich 6 ist, mit wenigstens einem Alkalihydroxid und einem Alkanol der Formel ROH (II), in der R 1 bis 12 Kohlenstoffatome umfaßt, in Gegenwart eines Sequestrierungsmittels der allgemeinen Formel

$$N—[CHR_1—CHR_2—O—(CHR_3—CHR_4—O—)_n—R_5]_3. \qquad (III)$$

umsetzt, in der n eine ganze Zahl größer oder gleich 0 und kleiner oder gleich 10 ($0 \leq n \leq 10$) ist, $R_1$, $R_2$, $R_3$, $R_4$ gleich oder verschieden ein Wasserstoffatom oder ein Alkylradikal mit 1 bis 4 Kohlenstoffatom bedeuten $R_5$ einen Alkyl- oder Cycloalkylrest mit 1 bis 12 Kohlenstoffatomen, einen Phenylrest oder einen Rest der Formel : $-C_mH_{2m}-Ø$ oder $C_mH_{2m+1}-Ø-$ bedeutet und m eine ganze Zahl zwischen 1 und 12 ist.

2. Verfahren nach Anspruch 1, dadurch gekennzeichnet, daß in der Formel (III) des Sequestrierungsmittels $R_1$, $R_2$, $R_3$ und $R_4$ gleich oder verschieden ein Wasserstoffatom oder einen Methylrest bedeuten.

3. Verfahren nach Anspruch 1, dadurch gekennzeichnet, daß in der Formel (III) des Sequestrierungsmittels n größer oder gleich 0 und kleiner oder gleich 6 ist.

4. Verfahren nach Anspruch 1, dadurch gekennzeichnet, daß in der Formel (III) des Sequestrierungsmittels $R_5$ einen Alkylrest mit 1 bis 4 Kohlenstoffatomen bedeutet.

5. Verfahren nach Anspruch 1, dadurch gekennzeichnet, daß in der Formel (III) des Sequestrierungsmittels $R_1$, $R_2$, $R_3$ und $R_4$ identisch oder verschieden ein Wasserstoffatom oder einen Metylrest bedeuten, n eine ganze Zahl größer oder gleich 0 und kleiner oder gleich 6 und $R_5$ eine Alkylrest mit 1 bis 4 Kohlenstoffatomen ist.

6. Verfahren nach Anspruch 5, dadurch gekennzeichnet, daß das Sequestrierungsmittel der Formel (III) das Tris(3,6-dioxyheptyl)amin der Formel :

$$N-(CH_2-CH_2-O-CH_2-CH_2-O-CH_3)_3$$

ist.

7. Verfahren nach Anspruch 5, dadurch gekennzeichnet, daß das Sequestrierungsmittel der Formel (III) das Tris(3,6,9-trioxadecyl) amin ist.

8. Verfahren nach Anspruch 1, dadurch gekennzeichnet, daß das Alkanol aus der Gruppe von Methanol, Äthanol, Isopropanol, den Butanolen, Cyclohexanol und Octanol gewählt wird.

9. Verfahren nach Anspruch 1, dadurch gekennzeichnet, daß das Polychlorbenzol der Formel (I) aus der Gruppe von Orthodichlorbenzol, Metadichlorbenzol, Paradichlorbenzol, 1,2,4-Trichlorbenzol, 1,2,3-Trichlorbenzol, 1,3,5-Trichlorbenzol, Tetrachlorbenzol, Pentachlorbenzol und Hexachlorbenzol gewählt ist.

10. Verfahren nach Anspruch 1, dadurch gekennzeichnet, daß das Alkalihydroxid Natrium- oder Kaliumhydroxid ist.

11. Verfahren nach Anspruch 1, dadurch gekennzeichnet, daß man ein Gemisch aus Natrium- und Kaliumhydroxid verwendet, das wenigstens 50 % Kaliumhydroxid enthält.

12. Verfahren nach Anspruch 1, dadurch gekennzeichnet, daß man die Verbindung (I) und das Alkalihydroxid in solchen Mengen umsetzt, daß das Molverhältnis des Alkalihydroxids zur Verbindung (I) 0,5 bis 3 beträgt.

13. Verfahren nach Anspruch 12, dadurch gekennzeichnet, daß das Molverhältnis zwischen 0,8 und 2,5 liegt.

14. Verfahren nach Anspruch 1, dadurch gekennzeichnet, daß man den Alkohol der Formel (I) in solcher Menge umsetzt, daß das Molverhältnis des Alkohols der Formel (I) zum Alkalihydroxid zwischen 1 und 15 liegt.

15. Verfahren nach Anspruch 14, dadurch gekennzeichnet, daß das Molverhältnis zwischen 6 und 8 liegt.

16. Verfahren nach einem der vorangehenden Ansprüche, dadurch gekennzeichnet, daß man bei einer Temperatur zwischen 80 und 250 °C arbeitet.